# EUROPEAN PATENT APPLICATION

(11) **EP 0 806 209 A2**
(43) Date of publication of application: **12.11.1997**
(21) Application number: 97301735.3
(22) Date of filing: 14.03.1997
(51) Int. Cl.: A61L 9/22, F24F 6/00

(54) **Method and apparatus for producing hydroxyl ion containing air for humidification**

(30) Priority: 14.03.1996 JP 84497/96
(71) Applicant: Kubo, Tetsujiro, Tokyo 150 (JP)
(72) Inventor: Kubo, Tetsujiro, Tokyo 150 (JP)
(74) Representative: Browne, Robin Forsythe, Dr.

(57) **Abstract**

An apparatus and method for producing hydroxyl minus ion - containing air with the function of humidification, by which the humidity of the air is increased and stabilized and which can be always used without limitation of season, climate and area. According to preferable embodiment, the apparatus comprises fibrous humidifying means by water vaporization, blowing means for directly compulsory blowing air to the humidifying means and a tourmaline carrier located at the down wind of humidifying means so that they can contact to the air. The tourmaline carrier comprises tourmaline particles having the average size of 0.3 to 3 microns and air contacting means, which is formed by mixing the tourmaline particles, which has the volume intrinsic electrical resistance of 10⁷ to 10¹⁰ Ω. cm, and which has the large surface area and which is breathable due to its structure such as sponge-shaped structure, honeycomb-shaped structure, aggregate-shaped structure of particles.

## Description

### BACKGROUND OF THE INVENTION

### 1. Field of the Invention

An apparatus and method for producing hydroxyl minus ion - containing air with function of humidification in accordance with the present invention generally relate to those for reforming air in living environment wherein the minus ions (hydroxyl ions) contained in the reformed air is absorbed into a body through the body surface and by means of the respiration, offering useful effect in the field of health and medical treatment.

Therefore, the apparatus and method are utilized for reforming air in the rooms of hospitals, asylum for the aged, homes, offices and any other places. The resultant hydroxyl minus ion - containing air has a large deodorizing effect, adding to the above effect in the field of health and medical treatment.

### 2. Description of the Prior Art

More than 50 years ago, experts in many fields found that ions contained in air have various kinds of function by large scaled researches. Hence, there have been many researches related to methods and apparatuses for generating the useful minus ions.

The present inventors filed, on 1994 August 31, an invention (Japanese Patent Application Number 6-23045) related to the apparatus and method for producing "hydroxyl minus ion - containing air" by contacting, small aggregates each of which consists of water molecules and which form moisture in air, to numberless of micro electric fields generated by the electrodes of tourmalines carried by the tourmaline carner so that the water molecules of each small aggregate are partially ionized.

The present invention is based on the above prior invention. The basic technique of this prior invention is that the small aggregates, each of which consists of water molecules in the air and which are usually recognized as a moisture component, are ionized into plus and minus ions by utilizing the micro electrodes of the tourmalines. That is to say, the amount of moisture component has a large influence to the structure of the apparatus and the method for producing the hydroxyl minus ion - containing air with the function of humidification. Actually, during the application of the above prior invention, this was clearly proved by the present inventors through their research, experiments and execution tests.

In connection with this fact, as a treatment for solving the problem of the prior invention, some points which should be improved in the prior invention and new matters which should be added to the prior invention are found clearly. This treatment offers the greatly improved and stabilized technical contents and effects, further, enlarged application field and operation over the above prior invention. Since this treatment requires large modification in the theory, construction, and operation, the inventors decided new patent application as the present invention.

The (relative) humidity of the air in our living environment vigorously changes throughout a year. In the pacific-side area of Japan, temperature is low and absolute humidity is also low outside in winter, additionally, in the room, the relative humidity is further low due to heating. Hence, humidifiers are commonly used in such climate condition of winter.

In this season, the concentration of small aggregate of water molecule serving as a moisture component in air is largely decreased, that is to say, the humidity is decreased to a great degree. Therefore, the efficiency of humidifier for producing the minus ions ions in accordance with the above prior invention is lowered so that its effect is decreased. Further, it is often expenenced that humidity changes not only as season changes, but also as climate changes in short period.

The temperature differs very much every zone in the world, in its tropical zone, subtropical zone, temperate zone, and frigid zone. Likely, the humidity differs very much every zone. Accordingly, the effect in the application of the apparatus of the above prior invention also differs very much every zone. The solution for this problem is the important research theme of the technique in the prior invention and inevitable for utilizing this apparatus. In the view of importance of this solution, the present inventors have performed various theoretical researches and experiments for about one year in order to carry out actual execution tests and solve this problem. As a result, the present invention is attained.

As stated above, the apparatus and method of the present invention can be always applied and stable effect can be obtained in regardless to season, climate, and zone where they are used. That is to say, the present invention improves fundamentally the technique of the prior invention, increases the effect obtained by the prior invention, and enlarges the application field of this technique drastically.

According to the apparatus and method in the prior invention, the aggregates each of which consists of water molecules in the air and which have been forced to pass by blowing means using a fan, are passed through the "air contact reaction unit" together with the air. During this passing, from each small aggregate of water molecules (the aggregate is said to consist of about three molecules), which is recognized as the moisture component, the water molecules are partially ionized in the numberless of micro electrical fields formed by the electrodes of the refined tourmalines careered by the tourmaline carrier. Thus, the "hydroxyl minus ion" - containing air is produced so as to be diffused in space. This operation is carried out repeatedly.

### SUMMARY OF THE INVENTION

On the other hand, in the present invention, the passed air is humidified just before passed through the "air contact reaction unit" so as to have relative humidity of 40 to 60 % when it passes this unit. For this, the "humidification unit" is located so that the air can be passed through it just before introduced into the "air contact reaction unit". Further, the amount of the passed air is adjusted as desired.

There are many types of method and means for humidifying air. They are generally divided in two types; one type includes those by vaporization and the other type includes those by sprayed steam. In the both cases, they are called as humidifiers. However, the condition of water forming the moisture by one type differs clearly from that by the other type. It is sure that original water is in liquid phase in the both cases. Then, the moisture component produced by using the vaporization is not in the state of liquid phase but "gas phase" so as to have material property of gas. Precisely, the small aggregates each consisting of about three water molecules are diffused, mixed and floated in the air molecules (oxygen, nitrogen, carbon dioxide gas and the like) in air. The size of each aggregate is substantially same as the size of each air molecule. It was disclosed that the particle size of the aggregate is approximately 6 to 7 x 10⁻⁸ cm (Static Electricity Handbook published by Polymer Society in 1989 October).

The water in the gas phase of this moisture component is aggregated so as to be water in liquid phase for forming water droplets or water screens when the humidity is decreased to a point. This point is called as dew point humidity. The particle shaped water in liquid phase is called as the water droplet. The particle size of water droplet of mist is 0.01 mm, while the water droplet of rain is in the range of 0.5 to 3 mm. It is therefore said that the one water droplet of rain corresponds to 1,000,000 water droplets of mist.

In Japan, so-called ultrasonic humidifiers are often used. In the ultrasonic humidifier, each water droplet of water in liquid phase is broken by ultrasonic pressure vibration and the resultant small water droplet in the state of steam is sprayed into the air. On the other hand, in the humidifier by water vaporization the water in liquid phase is volatilized and vaporized. Precisely, the small water droplet obtained in the ultrasonic humidifier is a water particle in liquid phase formed by braking the water droplet with mechanical force. Accordingly, the size ol the particle in the state of steam is different from that obtained by water vaporization. In the case of ultrasonic humidifier, the steam is visible in the state of white mist just after spraying.

In the case of ultrasonic humidifier, unlike the humidifier by vaporization, the impurities are often contained, sometimes bacteria or virus are contaminated or dissolved in water particles in the slate of mist. After each water droplet in the state of mist is secondly volatilized (vaporized), the moisture component in the gas phase is obtained. On this volatilization, the impurities contaminated in the water particles are separated from the water molecules so as to become impurities in air, which causes sanitary problem. Additionally, the water droplets are sometimes not secondly vaporized and attached to the surface of the units of apparatus as they are, which causes "wetting". After that, they are volatilized (vaporized) so as to become moisture component in air.

Among the above two kinds of means for humidifying, as the method used in the present invention, the means by water vaporization should be selected. The means, like the ultrasonic humidifier, where the water is used in the state of steam is unsuitable, because by such means, the wetting would be caused on the surface of tourmaline carrier in the air contact reaction unit, resulting in disturbing electrode reaction.

In the case of humidifying by water vaporization, the amount of water volatilized by this vaporization is proportion to the difference between humidity of the passed air when humidified at the humidification unit and saturated humidity at the temperature of the air passed through the humidifation unit. Additionally, this amount generally depends on the absorbing ability of passed air which is humidified, the absorbing ability of humidifying material in the humidification unit, and efficiency and contact time between the passed air and humidifying material. When the passed air is dry and has low humidity, the degree of humidification of the air is increased. On the other hand, when the passed air has high humidity, the humidification degree is decreased.

Therefore, humidity conditioning is performed according to the humidity of the passed air. The humidification degree can be controlled by adjusting the volume (speed) of compulsory blowing air. It is common that comfortable temperature and humidity in the living environment is 15 to 20 °C and 40 to 60 %, respectively. It is required to consider the structure and adjusting operation of this apparatus in order to include the temperature and humidity in such ranges.

It is clear from the above explanation that, in summer, when humidity is high and temperature is high, the moisture component can be partially changed to the hydroxyl ions by this apparatus. These hydroxyl ions accelerate the vaporization of water (sweat) attached to the surface of skin and under wear of each user. Then, the user feels comfortable, because the water's temperature is decreased by expensing vaporization heat. This effect is similar to the effect of cooling fan. On the other hand, in winter, when air is dry and humidity is low, this apparatus can be used for humidifying. Additionally, since this humidifying is carried out by vaporization, air is not contaminated.

In the apparatus and method for producing the hydroxyl minus ion - containing air with the function of humidification in accordance with the present invention, the ionization of not air itself but of water in the air is directed. This direction is basically the same as the above prior invention on which the present invention is based. Then, the present inventors paid attention to the fact that water is contained in air. The water contained in air is called as moisture whose degree is expressed by humidity. The water is floated in the form of small aggregates each consisting of few water molecules. The radius of the water molecule is 1.5 x 10⁻⁸ cm.

According to the apparatus and method in the above prior invention, the aggregates, each of which consists of water molecules in air and which has been forced to pass by blowing means using a fan, are passed through the "air contact reaction unit". On the passing, from each small aggregate of water molecules (it is said that the aggregate consists of about three molecules), which is recognized as the moisture component, the molecules are partially ionized in the numberless of micro electrical fields formed by the electrodes of the refined tourmalines carried by the tourmaline carrier. Thus, the "hydroxyl minus ion" - containing air is produced so as to be diffused in space. This operation is carried out repeatedly.

In the present invention, the apparatus and method of the pnor invention are totally utilized. However, according to the present invention, the passed air is humidified just before passed through the "air contact reaction unit". Concretely, the "humidification unit" is located so that the air is passed through it just before introduced into the "air contact reaction unit". Further, the amount of the passed air is adjusted as desired.

Now, the concrete construction of the present apparatus and method for producing the hydroxyl minus ion - containing air with the function of humidification is stated closely. In accordance with one aspect of the present invention, the apparatus for producing the hydroxyl minus ion - containing air with the function of humidification has the following construction. This apparatus comprises the humidifying means by water vaporization. Then, this apparatus comprises the tourmaline carrier, which is provided so as to be located at the down wind of the humidifying means. Further, this apparatus comprises the blowing means, which directly blows air to the tourmaline carrier and the above humidifying means by water vaporization so that the tourmaline carrier and the humidifying means can contact to the air.

The above tourmaline carrier comprises the tourmaline particles having the average size of 0.3 to 3 microns and the air contacting means which is formed by mixing the above tourmaline particles, which has the volume intrinsic electrical resistance of 10⁷ to 10¹⁰ Ω.cm, which has the large surface area and which is breathable due to its structure such as sponge-shaped structure, honeycomb-shaped structure, aggregate-shaped structure of particles, or the like.

Secondly, in accordance with another aspect of the present invention, the method for producing the hydroxyl minus ion - containing air with the function of humidification has the following construction. This method comprises directly blowing, with the blowing means, the air to the tourmaline carrier, which is located at the down wind of the humidifying means by water vaporization so that the tourmaline carrier can contact to the air. Thus, the water (moisture) in the air is ionized so that the hydroxyl ions can be produced.

The above tourmaline carrier comprises the tourmaline particles having the average size of 0.3 to 3 microns and the air contacting means which is formed by mixing the above tourmaline particles, which has the volume intrinsic electrical resistance of 10⁷ to 10¹⁰ Ω. cm, which has the large surface area and which is breathable due to its structure such as sponge-shaped structure, honeycomb-shaped structure, aggregate-shaped structure of particles, or the like.

Thirdly, in accordance with still another aspect of the present invention, the apparatus for producing the hydroxyl minus ion - containing air with the function of humidification has the following construction. This apparatus has the same construction as that of the first mentioned apparatus except one matter explained below. Accordingly, the whole of explanation of the construction of the above apparatus is used and one matter is added to it. Precisely, in this apparatus, the tourmaline carrier is specified as the carrier manufactured with fiber, elastomer, ceramic or plastic.

At last, in accordance with further another aspect of the present invention, the method for producing the hydroxyl minus ion - containing air with the function of humidification has the following construction. This method has the same construction as that of the secondly mentioned method except one matter explained below. Accordingly, the whole of explanation of the construction of the above method is used and one matter is added to it. Precisely, in this method, the tourmaline carrier is specified as the carner manufactured with fiber, elastomer, ceramic or plastic.

### BRIEF DESCRIPTION OF THE DRAWINGS

The object and advantages of the invention will become understood from the following detailed description of preferred embodiments thereof in connection with the accompanying drawings in which:
Fig. 1 is a front sectional view showing an embodiment of the apparatus for producing hydroxyl minus ion - containing air with the function of humidification in accordance with the present invention;
Fig. 2 is a front sectional view showing another embodiment of the apparatus in accordance with the present invention;
Fi g. 3 is a front sectional view showing still another embodiment of the apparatus in accordance with the present invention;
Fig. 4 is a partially enlarged plan view of the part of tourmaline, carrier of the apparatus in accordance with the present invention;
Fig. 5 is a front sectional view showing further another embodiment of the apparatus in accordance with the present invention;
Fig. 6 is a front sectional view showing still further another embodiment of the apparatus in accordance with the present invention;
Fig. 7 is a temperature graph showing the result of measurement using the apparatus according to the present invention;
Fig. 8 is a humidity graph showing the result of measurement using the apparatus according to the present invention;
Fig. 9 is a numerical graph showing the temperature of Table 1;
Fig. 10 is a numerical graph showing the humidity of Table 1;
Fig.11 is a numerical graph showing the temperature of Table 2;

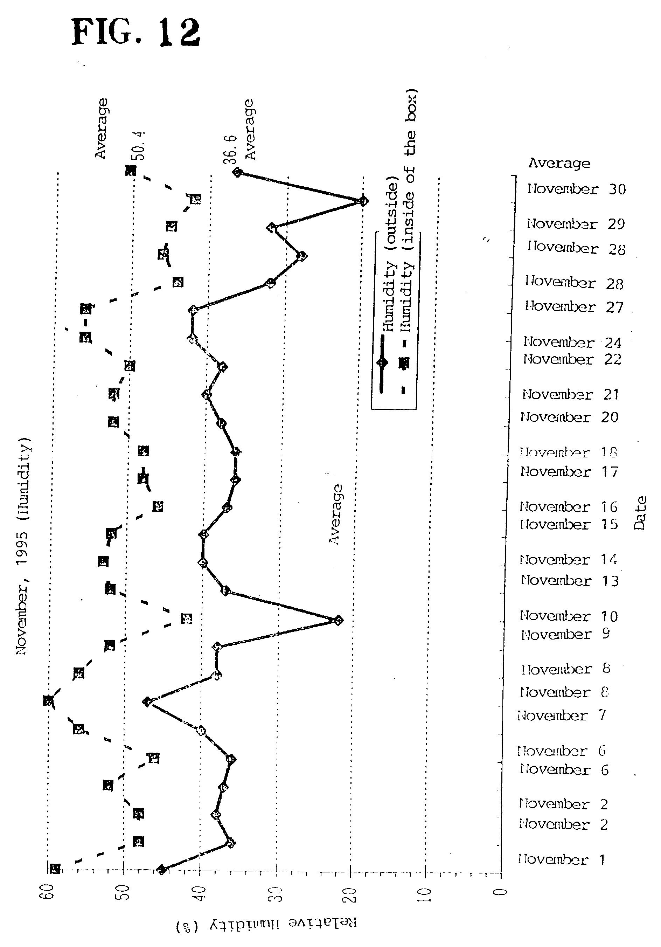

Fig. 12 is a numerical graph showing the humidity of Table 2;

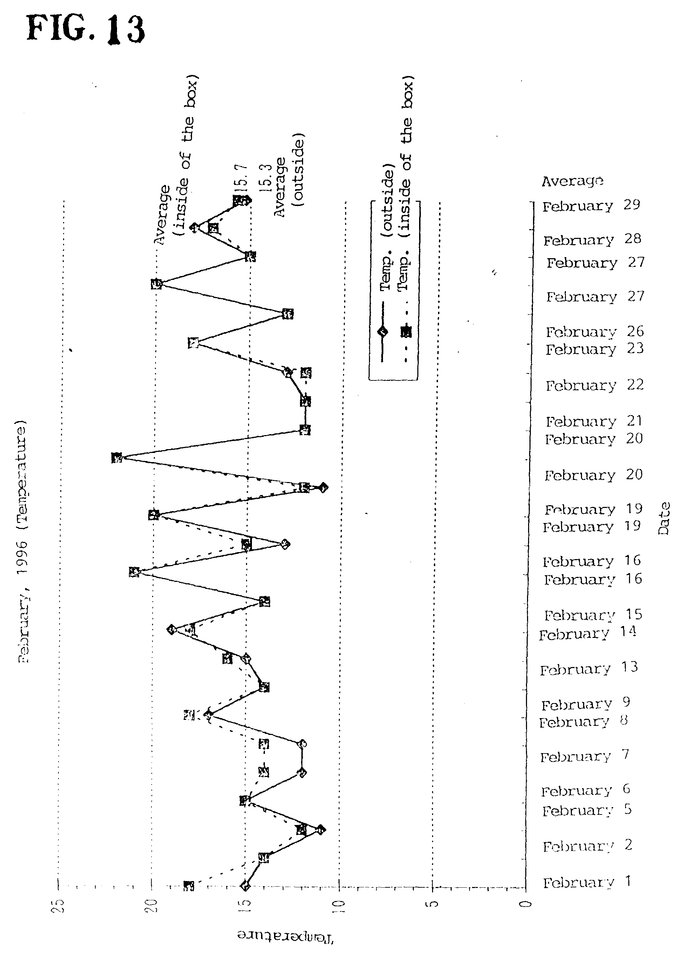

Fig. 13 is a numerical graph showing the temperature of Table 3; and

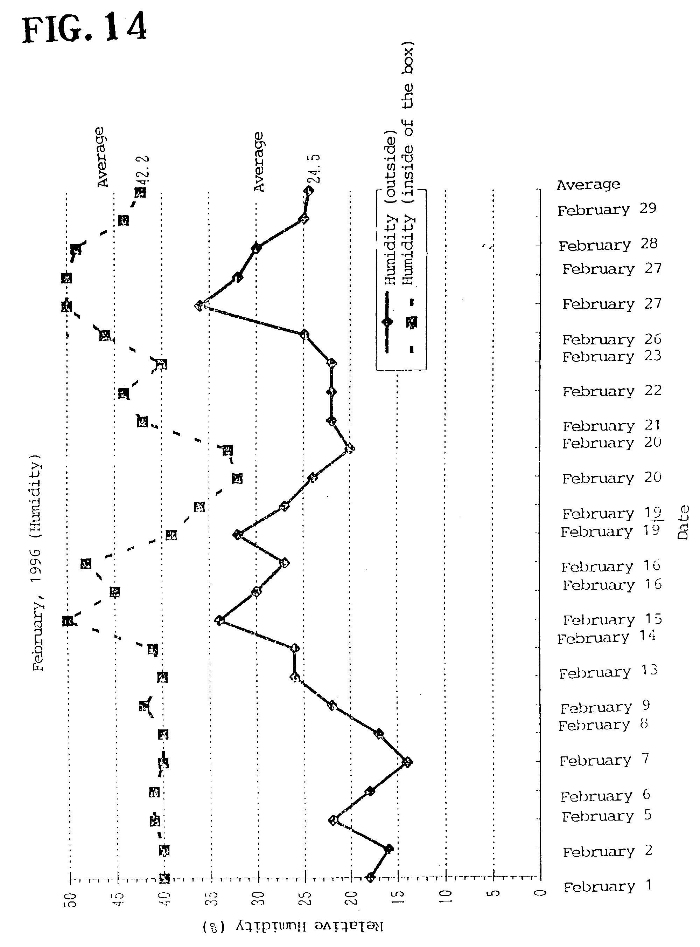

Fig. 14 is a numerical graph showing the humidity of Table 3.

### DETAILED DESCRIPTION OF THE PREFERRED EMBODIMENT

Now, the embodiments of apparatus and method for producing the hydroxyl minus ion - containing air with the function of humidification in accordance with the present invention are explained. First, in this apparatus and method, humidifying means by water vaporization is used. With this means, water is soaked into a fabric and the like and blowing is occurred there. Next, a tourmaline carrier is used, which is manufactured with a substance being breathable such as fabric. This tourmaline carrier is located down wind of the above humidifying means by water vaporization. Then, compulsory blowing means such as an electric fan and the like is used. This means directly forces the air to blow to the tourmaline carrier and the humidifying means by water vaporization so that the tourmaline carrier and the humidifying,,means can contact to the air. This blowing means may be located either up wind or down wind of the above humidifying means and tourmaline carrier or may be located at the intermediate between the above humidifying means and tourmaline carrier.

This tourmaline carrier comprises the tourmaline particles having the average size of 0.3 to 3 microns and the air contacting means which is formed by mixing the above tourmaline particles, which has the volume intrinsic electncal resistance of 10⁷ to 10¹⁰ Ω.cm, and which has the large surface area and which is breathable due to its structure such as sponge-shaped structure, honeycomb-shaped structure, aggregate-shaped structure of particles, or the like.

### Embodiment

Now, embodiments of the apparatus and method for producing hydroxyl minus ion - containing air with the function of humidification in accordance with the present invention are stated below. First, as for humidifying means and method thereof, a water tank is provided so as to be located adjacent to the apparatus. Since the water is used for humidifying passed air, the tank stores water in order to compensate the decrease of water. Then, this tank is preferably structured so that water can be easily supplied thereto. The water discharged from the tank (water tank) is absorbed through the under surface of a humidification mat (or fabric) and used for humidifying the air which has been blown by a fan. The tank is configured so that water can be always supplied thereto.

There are two types of absorbing material and manner of use. One type of absorbing material is a mat manufactured with a polymeric compound. This polymeric compound is called as a polymeric high-absorbing material and can absorb water of 10 to 100 times as much as its own weight. This mat has the constant thickness and is configured so as to contain sufficient amount of water and be highly breathable, for example the mat has a sponge-shaped structure. By partially immersing the end of mat into the water tank, the water is absorbed on the total surface of the mat. Then, since the water absorbed on the surface of the mat is contacted with the air passed through small ventilating holes formed on the total surface of the mat, the water is vaporized whereby the humidity of the passed air is increased. The other type of absorbing material is a belt manufactured by a fabric which is considerably easy to be wet and highly breathable. The lower end of belt is immersed into the water tank with a roller. The lower end of belt is wet in the water tank, and by rotating slowly an upper roller, the lower end of belt is moved up to reach the upper roller. On moving up, air is contacted with the fabric for humidification.

In the above both types, it is important that the humidification unit is located so that the air is passed through it just before introduced into the contact reaction unit, which is produced with the tourmaline carrier. By this location, the air is passed through the contact reaction unit while it is humidified. In the same way as common air conditioner, at the inlet of contact reaction unit, a filter is provided so as to remove dusts in the air.

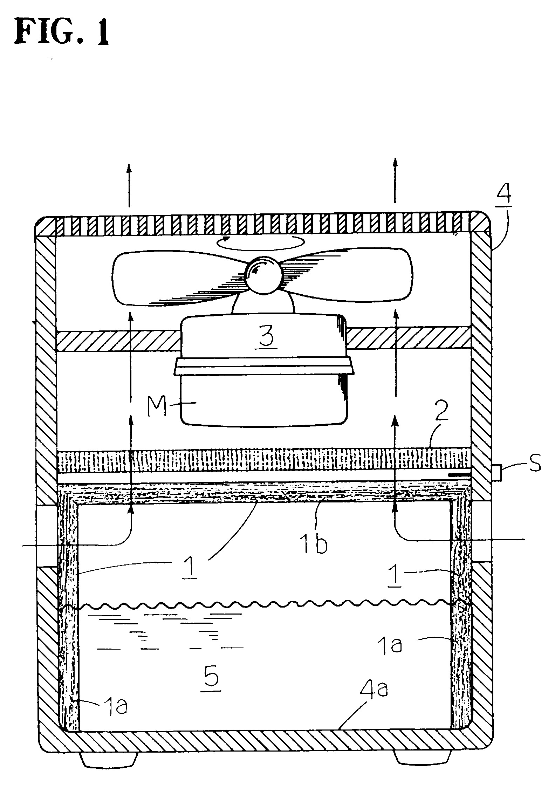

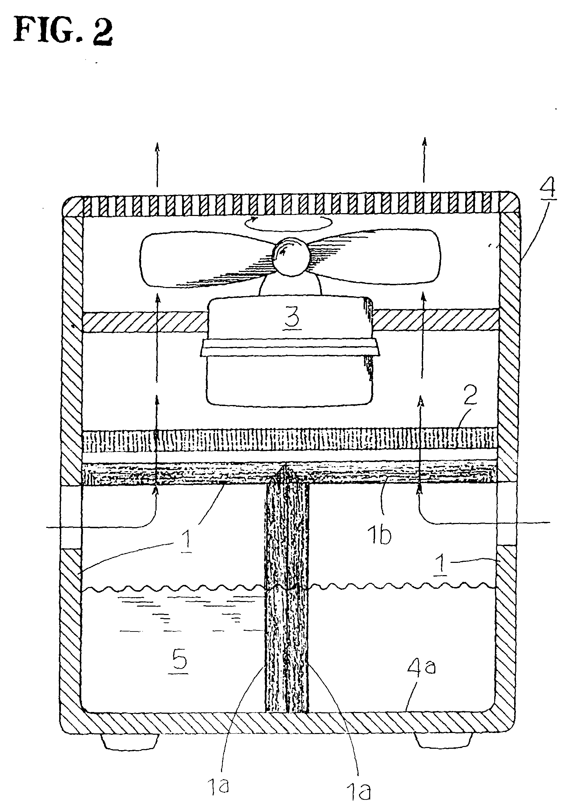

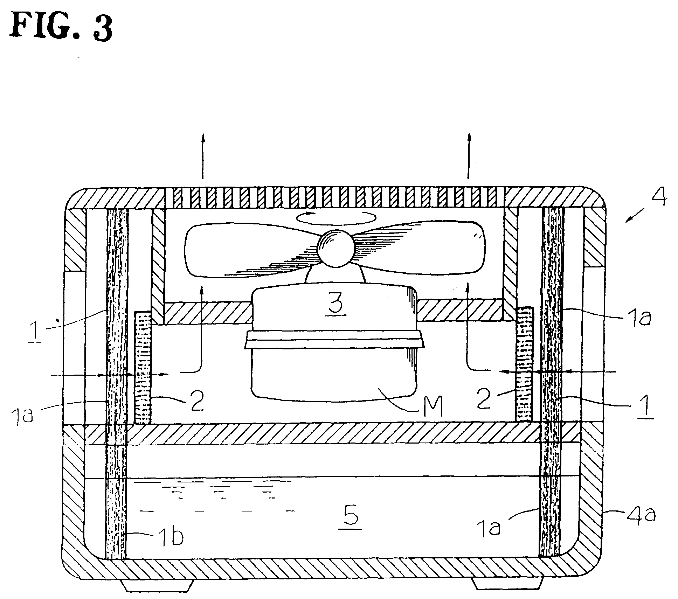

Now, the apparatus and method for producing the hydroxyl minus ion - containing air with the function of humidification in accordance with the present invention are stated below by way of their concrete embodiments in connection with the accompanying drawings. As shown in the front sectional view of Fig. 1, this apparatus comprises the humidifying means 1. In this humidifying means 1, the absorbing material manufactured with fiber, elastomer, ceramic, or plastic is immersed into water, and the air is passed through the absorbing material in order to vaporize the water. In Fig. 1, the immersed portion 1a of the humidifying means 1 is provided at the opposite sides of the humidification main body 1b. However, as shown in Fig. 2, the immersed portions la may be provided at the center of the humidification main body 1b. In the apparatuses shown in Figs. 1 and 2, the humidifying means 1 is provided vertically and the tourmaline carriers 2 stated below is provided horizontally. On the other hand, as shown in Fig. 3, the humidifying means 1 and the tourmaline carriers 2 may be provided vertically.

Next, the tourmaline carrier 2 comprising breathable material such as fabric is provided. The tourmaline carrier 2 is located down wind of the humidifying means 1 by water vaporization. Then, this apparatus comprises an electric fan 3 serving as a compulsory blowing means. The electric fan 3 directly forces air to blow to the tourmaline carrier 2 and the above humidifying means 1 by vaporization of water so that the tourmaline carrier and the humidifying means contact to the air. The electric fan 3 may be located either up wind or down wind of the above humidifying means 1 and tourmaline carrier 2 or it may be located at the intermediate between them. The above mentioned parts are installed in a frame body 4. Then, when the apparatus is operated, the desired amount of water 5 is supplied to the water tank 4a which defines the lower portion of the frame body 4.

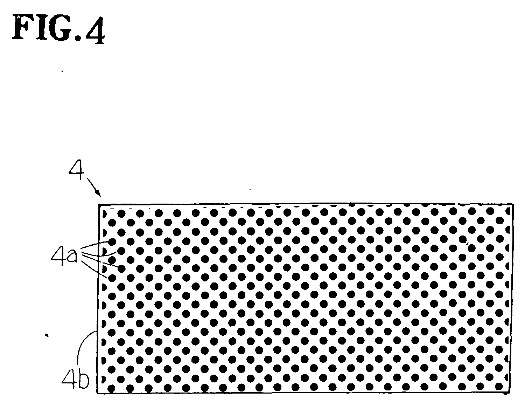

As shown in partially enlarged plan view of Fig. 4, the above mentioned tourmaline carner 2 comprises tourmaline particles 2a each having the particle size of 0.3 to 3 microns and a substance 2b which is formed by mixing these tourmaline particles 2a and which has the volume intrinsic electrical resistance of 10⁷ to 10¹⁰ Ω.cm. Then, this substance 2b has the large surface area and is breathable due to its structure such as sponge-shaped structure, honeycomb-shaped structure, aggregate-shaped structure of particles so as to be air contacting means 2b.

The electric fan 3 directly forces the air to blow to the above tourmaline carrier 2 which is located down wind of the humidifying means I by water vaporization so that the carrier 2 can contact to the air. By this contacting, the water molecules (moisture) in the air is ionized so that minus ions can be obtained.

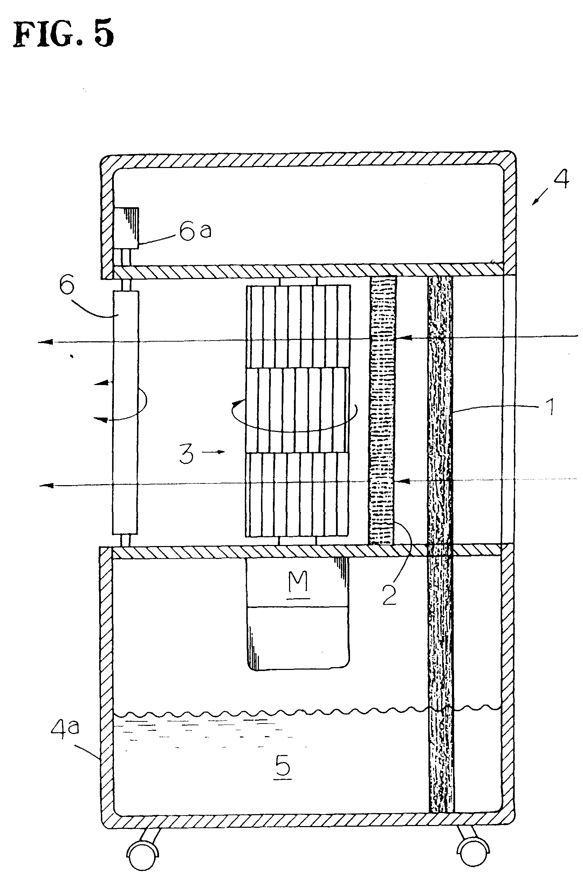

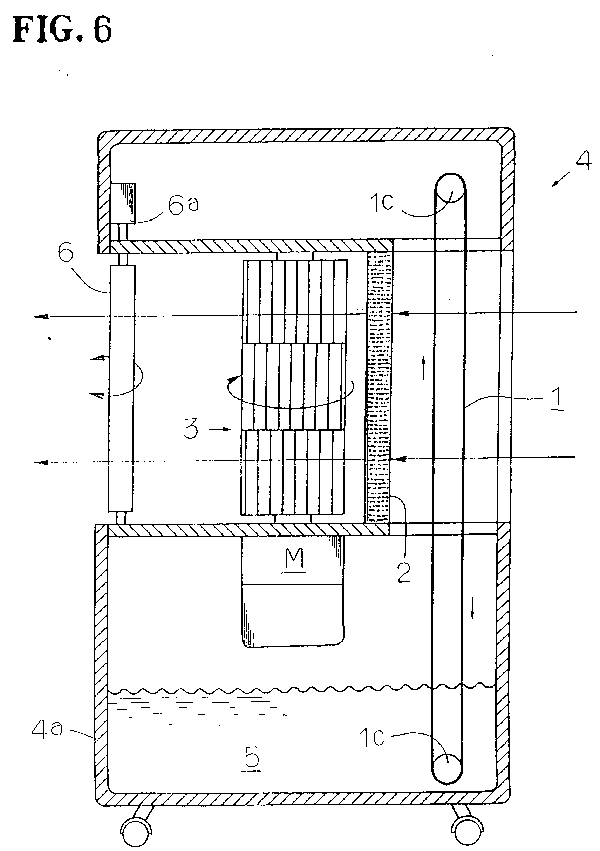

In the embodiments of Figs. 1 to 3, the air is blown in the upward direction. However, the embodiments of Figs 5 and 6, the air is blown in the horizontal direction. The embodiments of Figs. 1 to 3 are preferably used for small size apparatuses, while the embodiments of Figs 5 and 6 are preferably used for medium size apparatuses. In every embodiment except the embodiment of Fig. 6, the humidifying means 1 is a fix-type. In the embodiment of Fig. 6, the humidifying means 1 is a belt-type. The humidifying means 1 is rotated by a motor through a pair of rollers 1c. The humidifying means 1 is continuously immersed into the water tank 5 located below the humidifying means 1. In each of'the embodiments shown in Figs. 5 and 6, the electrical motor M of the electric fan 3 is covered with a water proof cover. This electric fan 3 is a rotary type. A rotating fin 6 is located down wind of the electric fan 3 and reciprocatively rotated with a motor 6a so that the air is fed out and diffused.

Now, the research for the effect of the present invention is explained.

Content: "Generator for hydroxyl minus ion - containing air" in accordance with the present invention was configured as an experimental model for the purpose of recognizing the following effect.

Object: This research was performed for recognizing the effect obtained by provision of a humidification filter. This humidification filter was provided so that air was humidified for increasing its humidity just before being passed through another filter by compulsory blowing. This filter used in the compulsory blowing was manufactured with rayon fiber (nonwoven fabric) and carried tourmaline crystals having permanent electrodes.

Apparatus used in research: The elements ol the apparatus used in this research is shown in Fig. 1. In this Figure, the present apparatus comprises a wooden frame body 4 and air contact reaction unit 2, which carries tourmaline crystals and which is manufactured with rayon nonwoven fabric. Then, the apparatus comprises an axial fan 3 which has 12 VDC, which is produced by Olix Co. and which is used for blowing. Further, the apparatus comprises a water container (water tank) 4a which is used for immersing the lower end of humidification mat.

Experiment: As the experimental models, vaporization type humidifier made in USA and that made in Swiss and vaporization type cooling fan humidifier by Toshiba Co. in Japan were used. The effect obtained by the provision of humidification unit was remarkable in every reformed experimental model.

In the case of experimental model in accordance with the above prior invention, the season with low humidity from 1994 December to 1995 February, there were some answers that deodorant effect against the smell caused by smoking was reduced (there were some answers, particularly in the room where there were many smokers, " The effect was reduced").

However, in the case of experimental model in accordance with the present invention, very good evaluations could be obtained as the following answers.
a. "Not only deodorant effect but also comfortable feeling of the air in the room where the apparatus was used could be obtained clearly."
b. "When only a cooler without the humidification unit was operated, specific uncomfortable feeling was caused."
c. "The feeling for the air in the room where the apparatus was used differed drastically from that in the room where the apparatus was not used."
d. "Pets (Cats) would not go out from the room where the apparatus was used."
e. "My appetite was increased." "I was not tired in the room where the apparatus is used." and "I could sleep well."
f. "I want that the apparatus can be soon available in market."

As stated above, the very good evaluations could be obtained. Therefore, it is expected the apparatuses are used in many places such as office, home, hospital, asylum for the aged, traffic (train, car, airplane) and the like.

When the apparatus and method for producing the hydroxyl minus ion - containing air in accordance with the present invention are applied, the following conditions are preferably required.
a. The presence of small aggregates each of which consists of few water molecules is important point of the present invention. Accordingly, the common vaporization type humidifier must be used in the apparatus and method for producing the hydroxyl minus ion - containing air. The ultrasonic spray type is not available, because, if humidifier of this type is used, the tourmaline carner in the air reaction unit would be wet, whereby electrode reaction would not be occurred.
b. The apparatus should be configured such that after the air is passed through the humidification unit, the resultant moisture component can be passed through the air reaction unit as it is. Then, the minus ions (hydroxyl ions) produced in the air reaction unit are mixed into the moisture component in the air. It should be noted that although the minus ions are unstable in the liquid, they are stable in air, because the air serves as an isolator. But when the minus ions are absorbed into water or sweat on the skin surface of a human body, they work as surface active agents.
c. It is generally said that comfortable humidity is 40 to 60 %. However, in the hydroxyl minus ion - containing air, these ions are adsorbed and absorbed into the water on the surface of skin and under wear of each user so that the water vaporization is accelerated. Since vaporization heat is expensed, the user can feel that the humidity is decreased and the climate is comfortable even in summer with high humidity. In this situation, the hydroxyl ions function as not minus ions in the air but surface active agents for the water.

Now, the experiment is explained concretely.

### 1. Method of Experiment

(1) The experimental apparatus as shown in Fig. 1 was operated on a desk at the corner of a room having the area of 39.6 m².
(2) The room is closed except when a person goes in and out of the room.
(3) Cooler and heater are used as usual.

### 2. Measurement

(1) The temperature and humidity (relative) were measured near the experimental apparatus (spaced from the apparatus with the distance of 50 cm to 1m) at the same height as that of the apparatus. At the same time, the temperature and humidity of the air which was passed through the humidification unit in the frame body just before passed through the air contact unit were measured with the meter of temperature and humidity provided on the wall of the frame body.
(2) The measurements were earned out about 10 a.m. and more than 20 days of every month from 1995 September to 1996 February at a city located on the pacific side of Japan.
(3) The results of these measurements in every month were indicated graphically and the average values were calculated.

### 3. Rayon Fiber Tourmaline Carrier

(1) Component of the rayon fiber was 66.5 wt % of rayon, 2.5 wt% of tourmaline (0.4 µ of average particle size) and 30 wt % of polyester (total; 100%). The rayon fiber has 7 denier (7 g / 9000 m).
(2) The nonwoven fabric (75 g / 1 m²) formed by the above polyester and tourmaline rayon was used in the tourmaline carrier.
(3) As the rough tourmaline, ores produced in China were used. Each ore has the strength electrode factor disclosed in the patent specification of Japanese Patent Application Number 5-36234. These ores were ground to powders each having the avenge size of 3 µ by Peking Metallurgy Industry Steel Research Center. After, they were imported, they were ground to particles each having the average size of 0.4 µ.

### 4. Air Contact Reaction Unit

(1) As the material for passing the air used in the air contact reaction unit, four sheets of nonwoven fabric mats were used, each of which has the area size of 15 cm x 15 cm and the thickness of 3 cm.
(2) The total weight of nonwoven fabric mats was 75 g / m² x 0.15 m x 0.15 m x 4 sheets = 75 g x 0.09 m² = 6.75 g

### 5. Result of Measurement

The temperature and humidity out side of the apparatus used in the experiment were measured. Then, the temperature and humidity of the humidified air in the frame body of the apparatus, before being introduced into the air reaction filter, were measured. From these measurements, average values were obtained every month, as shown in the following table.

| | outside of the frame body | in the frame body, just after the humidification |
|---|---|---|
| September | 59.1 % (24.9°C) | 66.0 % (22.0°C) |
| October | 48.1 % (22.3 °C) | 61.3 % (20.9 °C) |
| November | 36.6 % (16.4 °C) | 50.4 % (16.2 °C) |
| December | 29.4 % (13.5 °C) | 42.6 % (13.5 °C) |
| January | 27.8 % (15.3 °C) | 44.1 % (16.2 °C) |
| February | 24.4 % (15.1 °C) | 42.1 % (15.6 °C) |

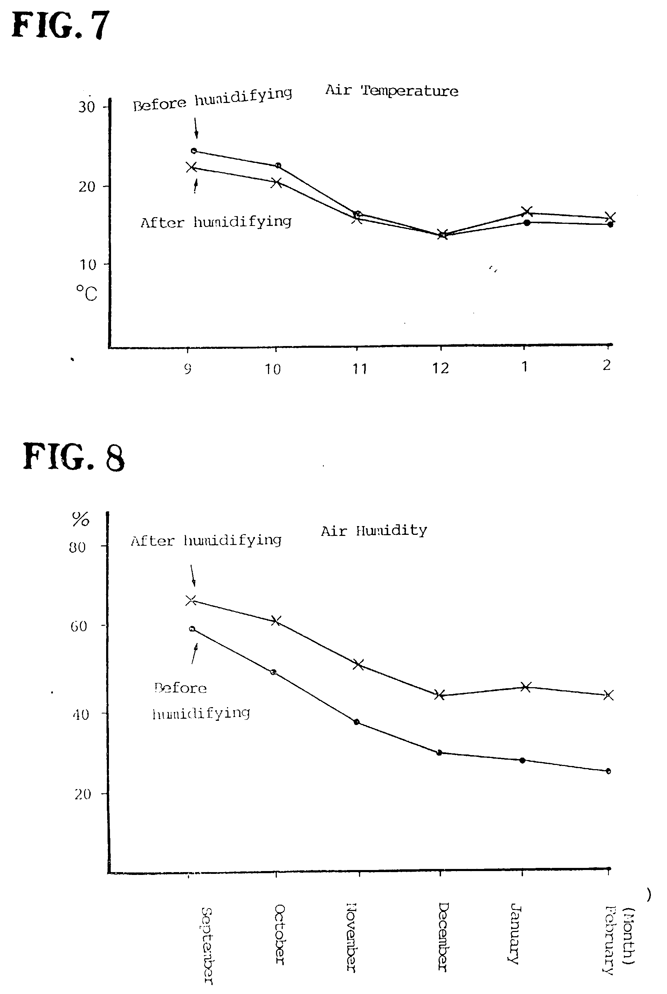

The above result is shown graphically in Figs. 7 and 8.

### 6. Summary of Result

(1) The average humidity in the room varies every month from Septembcr to February in the wide range of about 59 to 24 %.
(2) By provision of humidification unit, the average humidity is increased and stabilized in the range of about 66 to 42 %.
(3) From the fact that the average humidity is stabilized in the range of 66 to 42 % by the apparatus and method, it is clear that the present invention for producing hydroxyl minus ions in the air can offer the effect every season and every area.
(4) The temperature of the air after humidification in September was decreased from the temperature outside of the frame body by 2.9 °C. Then, in October and November, the temperatures were decreased by 1.4 and 0.2 °C, respectively. Further, in December, the temperature is not changed. In January and February, temperatures were increased by 0.9 and 0.5 °C. This means that in hot season such as September in Japan, the apparatus of the experiment offers the same effect as that of cooling fan, while, in winter season such as December, January, and February, the apparatus does not disturb the effect of heating.

The apparatus used in the experiment is shown in Fig. 1. The meter S for measuring temperature and humidity is configured so that the temperature and humidity of the air between the air humidification unit and air contact reaction unit is measured with its sensor and the result of measurement is indicated outside of the apparatus. The air introduced from the side of the apparatus is passed through the humidifying material's portions which are not immersed in the water and is crossed the humidifying material's horizontal portion so as to move upward.

Next, an equipment for measuring the concentration of ion contained in air is explained.

As an equipment for measuring the concentration of plus and minus ions contained in air, there is a measurement equipment developed by Kobe Electric Wave Co. (Kobe City in Japan). This equipment is based on a principle that the movement velocity of ion depends on the size of ion.

In the measurement of this experiment, the equipment of this kind of model number KSI - 900 was used. With this equipment, only ions each having the particle size of 0.0026 µm or smaller can be measured. As equipment of this kind, there are those of model number KSI - 1000 (for the particle size of 0.01 µm or smaller) and model number KSI - 2000 (for the particle size of 0. 1 µm or smaller). They are used for measunng the concentration of plus and minus ions and that of charged corpuscles contained in atmospheric air on the earth, in the purpose of research for the relation between the meteorological elements and the change of density of plus and minus ions contained in air.

Were measured the concentration of plus and minus ions each having the particle size of 0.0026 µm (2.6 x 10⁻⁷ cm) and smaller by utilizing the equipment of model number KSI - 900.

According to the literature (Static Electricity Handbook edited by Polymer Society), some data related to hydroxyl ions [(H₂O)ₙ + OH⁻] shown in the present invention is disclosed by Chapman in his report by way of measurement result of the size of water droplet. number of water molecules, and the mobility of water molecule.

| radius (cm) | number of molecules | mobility (cm²/V.cm) |
|---|---|---|
| 33 x 10⁻⁸ | 3 H₂O+H⁺ | 1.0 |
| 33 x 10⁻⁸ | 3 H₂O+OH⁻ | 0.95 |

In this report, it is inferred that three water molecules can carry two OH⁻.

Then, in an experiment with the apparatus for producing the hydroxyl minus ion - containing air, the numbers of plus and minus ions were measured by utilizing the above equipment on 9:30 a.m. In the room, there were three persons one of who was a smoker. One minute had passed after the smoker (spaced from the apparatus with about 1.5 m) smoked once, the number of plus ions per 1 cc of air increased to the value around 120. The numbers of plus and minus ions were measured, resulting in the following data.

| | minus ion (number / 1 cc air) | plus ion (number / 1 cc air) |
|---|---|---|
| apparatus is not operated | 28 to 38 | 30 to 33 |
| apparatus is operated | 80 to 85 | 28 to 38 |

(The maker that produces the measurement equipments of this kind reported, as the measurement result, the change of density of plus and minus ions in atmospheric air on ocean after typhoon, with the equipment of model number KSI - 1000 (for particle size of 0.01µm or smaller). According to this report, average number of plus and minus ions is about 100/1 cc air, throughout one day.)

In the above experiment with the apparatus for producing the hydroxyl minus ion - containing air in accordance with the present invention, the change of number of plus and minus ions produced with the minus ion - generator was researched.

As shown in the above explanation, the following results were obtained.
(1) When the generator was operated, the number of minus ions is 80 to 85, while the number of plus ions is 28 to 38.
(2) Before the generator was operated, the number of minus ions is 28 to 38, while the number of plus ions is 30 to 33.
(3) Within one minute after the smoker (spaced from the apparatus with about 1.5 m) smoked once, the number of plus ions increased to the value around 120.

The data obtained above is shown in the companied Table 1 to 3. Table 1 is the data of 1995 September, Table 2 is the data of 1995 November and Table 3 is the data of 1996 February.

The temperature and humidity of air outside the apparatus and in the frame work of the apparatus were measured in 1995 September, 1995 November and 1996 February, respectively. These measurement results are indicated in Tables 1, 2, and 3, in this order.

**Table I**

| date | temparature (°C outside) | humidity (% outside) | temparature (°C in frame work) | humidity (% in frame work) |
|---|---|---|---|---|
| September 1 | 20 | 62 | 26 | 63 |
| September 6 | 21 | 52 | 22 | 56 |
| September 16 | 27 | 63 | 23 | 73.5 |
| September 18 | 27 | 59 | 20 | 69 |
| September 19 | 27 | 57 | 21.5 | 68 |
| September 20 | 27 | 58 | 22 | 66 |
| September 21 | 27 | 54 | 20 | 63 |
| September 22 | 27 | 62 | 21.5 | 63.5 |
| September 23 | 27 | 47.6 | 23 | 63 |
| September 24 | 24 | 67 | 23 | 69 |
| September 25 | 25 | 62 | 23 | 66 |
| September 26 | 23 | 56 | 21 | 66 |
| September 27 | 23 | 71 | 22.5 | 74 |
| September 29 | 24 | 66 | 23 | 66 |
| September 30 | 25 | 49 | 22 | 64 |
| | | | | |
| average | 24.9 | 59.0 | 22.2 | 66.0 |

The temperature and humidity of air outside the apparatus and in the frame work of the apparatuis were measured in 1995 September, 1995 November and 1996 February, respectively. These measurement results are indicated in Tables 1, 2 and 3, in this order.

**Table 2**

| date | temparature (°C outside) | humidity (%outside) | Temparature (°C in frame work) | humidity (%in frame work) |
|---|---|---|---|---|
| November 1 | 21 | 45 | 20 | 59 |
| November 2 | 16 | 36 | 16 | 48 |
| November 2 | 19 | 38 | 18 | 48 |
| November 6 | 16 | 37 | 16 | 52 |
| November 6 | 18 | 36 | 18 | 46 |
| November 7 | 18 | 40 | 18 | 56 |
| November 8 | 18 | 47 | 18 | 60 |
| November 8 | 20 | 38 | 20 | 56 |
| November 9 | 14 | 38 | 14 | 52 |
| November 10 | 20 | 22 | 19.5 | 42 |
| November 13 | 17 | 37 | 17 | 52 |
| November 14 | 17 | 40 | 16 | 53 |
| November 15 | 16 | 40 | 16 | 52 |
| November 16 | 13 | 37 | 12 | 46 |
| November 17 | 16 | 36 | 16 | 48 |
| November 18 | 15 | 36 | 16 | 48 |
| November 20 | 14 | 38 | 14 | 52 |
| November 21 | 15 | 40 | 16 | 52 |
| November 22 | 14 | 38 | 14 | 50 |
| November 24 | 16 | 42 | 16 | 56 |
| November 27 | 12 | 42 | 11 | 56 |
| November 28 | 11 | 32 | 11 | 44 |
| November 28 | 21 | 28 | 20 | 46 |
| November 29 | 13 | 32 | 13 | 45 |
| November 30 | 21 | 20 | 21 | 42 |
| average | 16.4 | 36.6 | 16.2 | 50.4 |

**Table 3**

| date | temparature (°C outside) | humidity (% outside) | temparature (°C in frame work) | humidity (% in frame work) |
|---|---|---|---|---|
| February 1 | 15 | 18 | 18 | 40 |
| February 2 | 14 | 16 | 14 | 40 |
| February 5 | 11 | 22 | 12 | 41 |
| February 6 | 15 | 18 | 15 | 41 |
| February 7 | 12 | 14 | 14 | 40 |
| February 8 | 12 | 17 | 14 | 40 |
| February 9 | 17 | 22 | 18 | 42 |
| February 13 | 14 | 26 | 14 | 40 |
| February 14 | 15 | 26 | 16 | 41 |
| February 15 | 19 | 34 | 18 | 50 |
| February 16 | 14 | 30 | 14 | 45 |
| February 16 | 21 | 27 | 21 | 48 |
| February 19 | 13 | 32 | 15 | 39 |
| February 19 | 20 | 27 | 20 | 36 |
| February 20 | 11 | 24 | 12 | 32 |
| February 20 | 22 | 20 | 22 | 33 |
| February 21 | 12 | 22 | 12 | 42 |
| February 22 | 12 | 22 | 12 | 44 |
| February 23 | 13 | 22 | 12 | 40 |
| February 26 | 18 | 25 | 18 | 46 |
| February 27 | 13 | 36 | 13 | 50 |
| February 27 | 20 | 32 | 20 | 50 |
| February 28 | 15 | 30 | 15 | 49 |
| February 29 | 18 | 25 | 17 | 44 |
| | | | | |
| average | 15.3 | 25.4 | 15.7 | 42.2 |

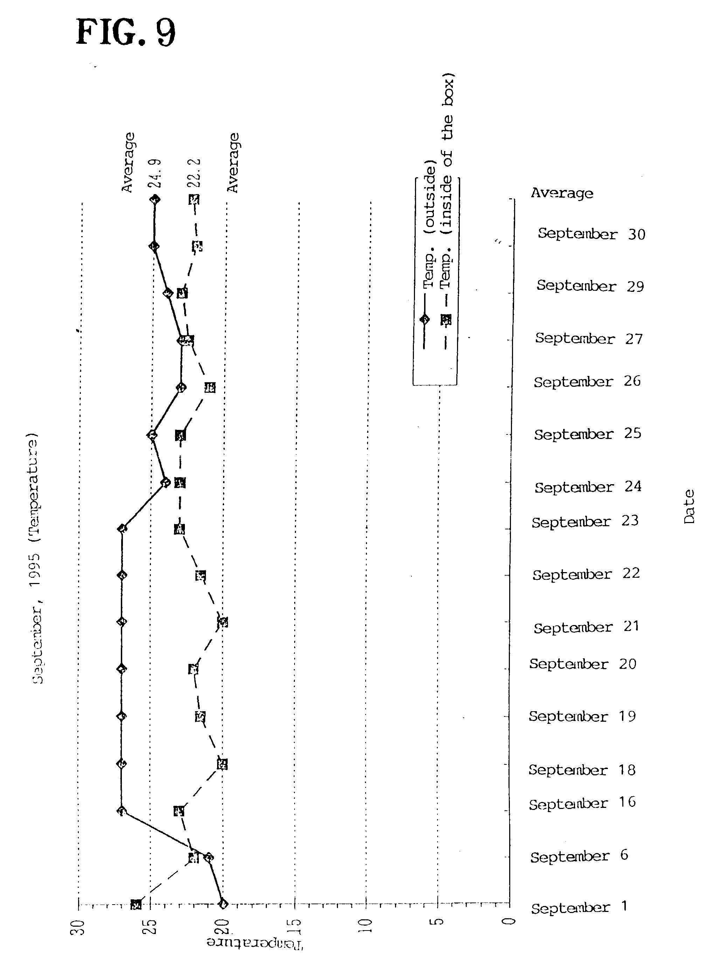

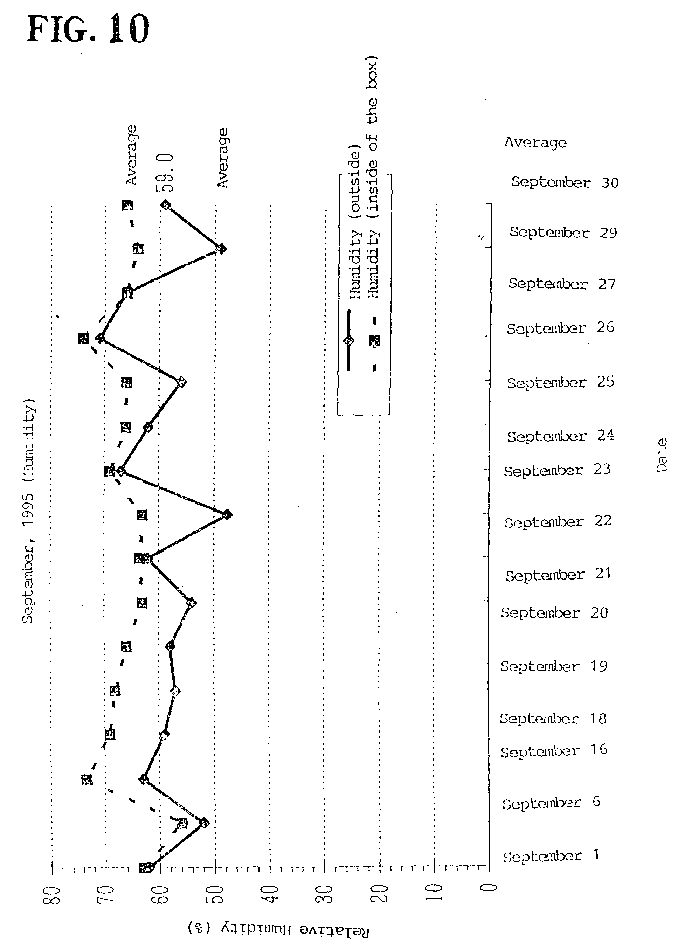

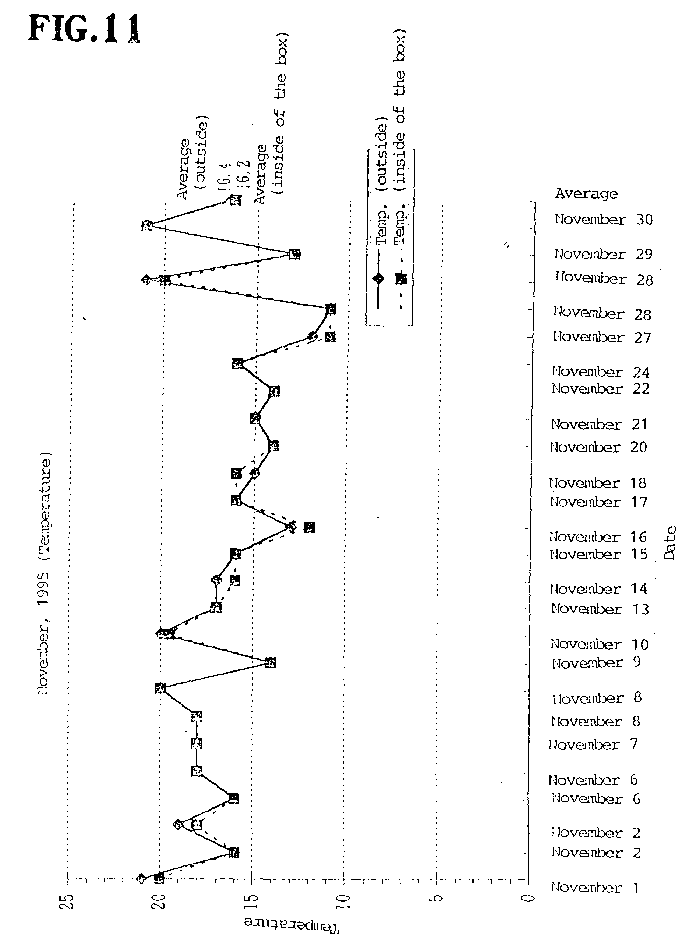

The numerical data of temperature and humidity of Tables 1, 2 and 3 are indicated graphically in Figs. 9, 10 and 11, respectively.

As stated above, the apparatus and method for producing the hydroxyl minus ion - containing air with the function of humidification in accordance with the present invention offer the great effect. Precisely, by the provision of humidification unit, the humidity of the air is increased to about 60 to 40%. Such increased and stabilized humidity 60 to 40% teaches that apparatus for generating minus ions in air can be always used throughout year without limitation of season, climate and area where the apparatus is used.

Particularly, the minus ions are absorbed and absorbed into the water on the surface of skin and under wear of each user, the water vaporization is accelerated and vaporization heat is expensed. Then, the user feels as if humidity was decreased comparing with actual humidity. Hence, even in summer with high humidity, the user can feel comfortable. That is to say, the apparatus and method offer useful effect like a cooling fan in summer, while they do not disturb the effect of heater.

## Claims

1. An apparatus for producing hydroxyl minus ion - containing air with function of humidification comprising:
humidifying means by water vaporization;
blowing means for directly compulsory blowing air to said humidifying means by water vaporization and a tourmaline carrier so that said tourmaline carrier and humidifying means can contact to said air; and
said tourmaline carrier located at the down wind of said humidifying means by water vaporization and comprising tourmaline particles having the average size of 0.3 to 3 microns and air contacting means, which is formed by mixing said tourmaline particles, which has the volume intrinsic electrical resistance of 10⁷ to 10¹⁰ Ω. cm, and which has the large surface area and which is breathable due to its structure such as sponge-shaped structure, honeycomb-shaped structure, aggregate-shaped structure of particles.

2. A method for producing hydroxyl minus ion - containing air with function of humidification comprising:
humidifying air with humidifying means by water vaporization;
directly compulsory blowing said air to a tourmaline carrier located down wind of said humidifying means by water vaporization so that they can contact to said air, said tourmaline carrier located at the down wind of said humidifying means by water vaporization and comprising tourmaline particles having the average size of 0.3 to 3 microns and air contacting means, which is formed by mixing said tourmaline particles, which has the volume intrinsic electrical resistance of 10⁷ to 10¹⁰ Ω. cm, and which has the large surface area and which is breathable due to its structure such as sponge-shaped structure, honeycomb-shaped structure, aggregate-shaped structure of particles; and
ionizing moisture contained said air for producing hydroxyl minus ions.

3. An apparatus for producing hydroxyl minus ion - containing air with function of humidification according to Claim 1 wherein said tourmaline carrier is manufactured with at least one material selected from the group of fiber, elastomer, ceramic and plastic.

4. A method for producing hydroxyl minus ion - containing air with function of humidification according to Claim 2 wherein said tourmaline carrier is manufactured with at least one material selected from the group of fiber, elastomer, ceramic and plastic.
